# FASCICULE DE BREVET EUROPEEN

(11) **EP 0 880 397 B1**
(45) Date de publication et mention de la délivrance du brevet: **20.11.2002**
(21) Numéro de dépôt: 97904504.4
(22) Date de dépôt: 06.02.1997
(51) Int. Cl.: B01D 59/34, G01R 33/26

(54) **INSTALLATION ET PROCEDE POUR LA PRODUCTION D'HELIUM-3 POLARISE EN PHASE VAPEUR, EN PARTICULIER POUR L'IMAGERIE PAR RMN**
ANLAGE UND VERFAHREN ZUR HERSTELLUNG VON IN DAMPFPHASE POLARISIERTEM HELIUM-3, INSBESONDERE FÜR NMR-BILDGEBUNG
APPARATUS AND METHOD FOR PRODUCING POLARISED VAPOUR-PHASE HELIUM-3, PARTICULARLY FOR NMR IMAGING

(30) Priorité: 16.02.1996 FR 9601973
(43) Date de publication de la demande: 02.12.1998
(73) Titulaire: CENTRE NATIONAL DE LA RECHERCHE SCIENTIFIQUE (CNRS), 75016 Paris (FR)
(72) Inventeur: NACHER, Jean-Pierre, F-94200 Ivry-sur-Seine (FR); TASTEVIN, Geneviève, F-92170 Vannes (FR)
(74) Mandataire: Breese, Pierre
(86) Numéro de dépôt international: FR9700240
(87) Numéro de publication internationale: WO97029836

(56) Documents cités:
- EP-A- 0 471 586
- WO-A-91/07668
- WO-A-95/27438
- FR-A- 2 598 518

## Description

La présente invention concerne une installation et procédé pour la production d'hélium-3 polarisé en-phase vapeur à forte pression, en particulier pour l'imagerie par RMN, ainsi que les applications d'une telle installation.

L'hélium-3 polarisé est un isotope connu pour les propriétés qui ont été mises en oeuvre en particulier pour la mesure de très faibles variations du champ magnétique terrestre, en particulier pour la réalisation de magnétomètres. Les méthodes de production d'hélium-3 polarisé dense de l'état de l'art sont relativement difficiles à mettre en oeuvre. Une première méthode dite "brute force" consiste à placer un échantillon d'hélium-3 dans un champ magnétique intense à basse température. Ce procédé n'est efficace que pour l'hélium-3 solide à une pression supérieure à 36 atmosphères, ou pour de l'hélium-3 très dilué dans de l'hélium-4 liquide, à une température de quelques millikelvin dans un champ magnétique de plusieurs teslas. Ces conditions posent des problèmes technologiques importants et ne permettent pas une production à des conditions économiques acceptables pour des applications nécessitant la production dans un délai raisonnablement court de volumes importants d'hélium-3 polarisé.

On a également proposé dans l'art antérieur la polarisation par échange de spin avec un gaz d'atomes alcalins orientés par pompage optique. Cette méthode est également très lente, car les collisions d'échange qui assurent le transfert de polarisation de l'alcalin à l'hélium sont peu efficaces et constituent un facteur limitant incontournable. Cette méthode nécessite une durée de 10 heures pour polariser 150 centimètres cube d'hélium à une pression de 10 bars et une polarisation de 10%.

On connaît encore un procédé qui consiste à comprimer l'hélium-3 gazeux polarisé par pompage optique à l'aide d'un compresseur à piston mobile. Un tel dispositif est très délicat à réaliser et à faire fonctionner. En effet, des précautions extrêmement rigoureuses doivent être prises pour éviter la relaxation pendant la phase de compression, lors du passage dans la pompe. Il convient en particulier d'éviter l'utilisation de tout lubrifiant susceptible de contaminer le gaz polarisé et de procéder à un choix des matériaux formant les parois de la pompe propres à réduire la relaxation des isotopes polarisés. La mise en oeuvre d'un tel procédé est possible pour des applications de recherche en laboratoire, mais ne permet pas actuellement de produire dans des conditions économiquement acceptables des quantités importantes d'hélium-3 fortement polarisé, dans des délais courts.

Le document WO-A-95 27 438 décrit un appareil pour délivrer un gaz polarisé comprenant une cellule ayant un point destiné à la rupture débouchant dans un cylindre à piston.

L'objet de la présente invention est de remédier aux inconvénients des procédés et installations de l'art antérieur en proposant un procédé permettant l'obtention rapide d'hélium-3 fortement polarisé sous forme de gaz dense, à une pression de l'ordre de la pression atmosphérique ou supérieure à la pression atmosphérique, à l'aide d'une installation d'un coût de réalisation faible et d'un fonctionnement simple.

A cet effet, l'invention concerne tout d'abord une installation pour la production d'hélium-3 polarisé en phase vapeur à forte pression, en particulier pour l'imagerie par RMN, l'installation comportant un moyen d'injection d'hélium dans une cellule de pompage optique, un moyen de liquéfaction du gaz polarisé provenant de la cellule de pompage optique et un réservoir pour le stockage d'hélium-3 polarisé en phase liquide caractérisé en ce que le réservoir d'accumulation est refroidissable par un système de refroidissement à une température ajustable entre une température d'accumulation Ta et une température d'évaporation Te et communique alternativement avec la cellule de pompage optique et avec un conduit d'évacuation de l'hélium-3 en phase gazeuse à forte pression.

L'installation selon l'invention fonctionne selon un principe purement cryogénique de compression, et ne met en oeuvre que des effets thermiques pour déplacer les atomes. On évite ainsi en particulier tous les problèmes découlant de l'utilisation d'une pompe pour la compression du gaz polarisé.

De préférence, la cellule de pompage optique communique avec la source d'hélium-3 par une vanne à débit réglable. Par rapport à la méthode d'échange de spins avec des atomes alcalins polarisés optiquement, l'avantage majeur est la rapidité de la production de l'orientation par pompage optique direct de l'hélium.

Selon un mode de réalisation préféré, la cellule de pompage optique comporte un moyen de détermination de la polarisation nucléaire du gaz.

Ce moyen, par exemple un dispositif propre à mesurer la polarisation du rayonnement de fluorescence émis par le mélange gazeux dans la cellule de pompage optique, permet de contrôler le taux de polarisation du mélange gazeux dans la cellule de pompage et d'ajuster certains réglages de l'installation pour maximaliser ce taux. L'installation comporte à cet effet des moyens d'ajustage du débit de la vanne d'alimentation de la cellule de pompage et/ou de la température Ta du réservoir d'accumulation en fonction de polarisation nucléaire dans la cellule de pompage optique.

Selon un mode de réalisation préféré, la cellule de pompage optique est excitée par un laser émettant une raie d'une longueur d'onde de 1083 nanomètres.

Par exemple, une diode laser d'une puissance de 50 milliwatts permet de préparer 100 centimètres cube d'un mélange contenant 10% d'hélium-3 sous une pression de 1,2 bars, en 15 minutes.

L'invention concerne également un procédé pour la production d'hélium-3 polarisé en phase vapeur consistant à injecter dans une cellule de pompage optique de l'hélium-3 et de stocker l'hélium-3 polarisé en phase liquide dans un réservoir de stockage caractérisé en ce que le gaz polarisé provenant de la cellule de pompage optique est recueilli dans un réservoir d'accumulation refroidi à une température ajustable entre une température Ta d'accumulation de l'hélium-3 et une température Te d'évaporation de l'hélium-3.

Avantageusement, on injecte dans la cellule de pompage optique un mélange d'hélium-3 et d'hélium-4. Il est apparu que l'utilisation d'un mélange, plutôt que de l'hélium-3 pur, a pour effet de prolonger la durée de maintien de la polarisation. De préférence ledit mélange contient entre 3 et 30% d'hélium-3.

Selon une variante avantageuse, on procède à une évaporation d'une partie de l'hélium polarisé contenu dans le réservoir. Cette variante se traduit par un enrichissement en hélium-3 polarisé dans le mélange gazeux polarisé produit par l'installation. On peut en outre recourir à des techniques d'enrichissement qui font appel à des effets thermodynamiques propres à l'hélium superfluide.

L'invention concerne encore l'application d'une installation conforme à ce qui précède à l'imagerie par RMN. On fait pénétrer dans un corps à analyser un mélange gazeux polarisé fourni par une installation selon l'invention et en ce que l'on procède à une imagerie par RMN dudit corps.

L'invention sera mieux comprise à la lecture de la description qui suit concernant un exemple non limitatif de réalisation et se référant aux dessins annexés où :

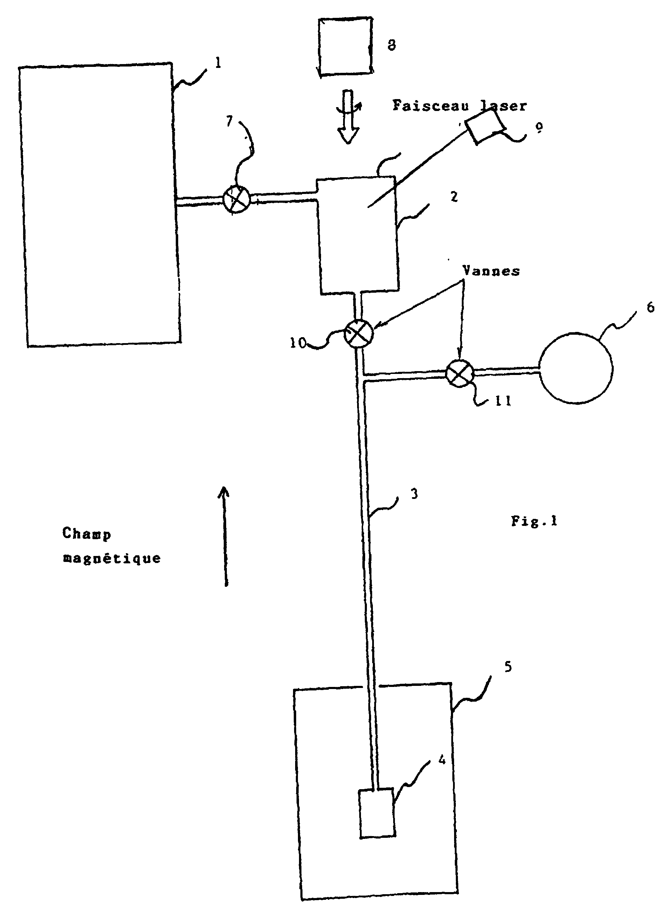

- la figure 1 représente une vue schématique de l'installation ;

La figure 1 représente une vue schématique d'une installation selon l'invention. Elle est constituée par un réservoir (1) contenant un mélange gazeux d'hélium-3 et d'hélium-4. Le mélange contient une proportion d'environ 10% d'hélium-3 pour 90% d'hélium-4.

Une vanne (7) permet d'injecter le mélange dans une cellule de pompage optique (2). Cette vanne (7) peut également être utilisée pour agir sur le débit d'injection du mélange dans la cellule (2).

La cellule de pompage (2) est un volume permettant la circulation des atomes entre le réservoir (1) et un conduit (3) débouchant dans un réservoir d'accumulation (4). La cellule (2) est réalisée en un matériau transparent non relaxant tel que verre, et est placé sur le trajet d'un faisceau laser à une longueur d'onde de 1083 nanomètres. Un polarimètre (9) permet d'analyser la lumière de fluorescence émise par le mélange contenu dans la cellule (2) et de déduire le taux de polarisation nucléaire. Cette mesure permet de contrôler l'efficacité du pompage optique. A titre de variante, il est possible de procéder à une mesure directe de l'aimantation du gaz dans la cellule (2) par RMN, magnétométrie, etc.

La sortie de la cellule (2) débouche dans une enceinte ou réservoir d'accumulation (4) présentant des parois en un matériau non relaxant. Cette enceinte d'accumulation (4) est refroidie par un système de refroidissement (5) permettant de réguler la température pour condenser ou évaporer le mélange ³He-⁴He contenu dans l'enceinte d'accumulation (4). Le système de refroidissement sera par exemple un réfrigérateur à hélium-4 pompé. Ce type de dispositif permet d'atteindre des températures de l'ordre de 0,9 K.

Pendant la phase d'accumulation, la température de l'enceinte (4) doit être inférieure à 1,8 K pour qu'à toute concentration la pression de vapeur soit inférieure à 10 Torr pour permettre le pompage optique. La température de l'enceinte (4) détermine directement la pression à l'intérieur de la cellule de pompage optique (2), et donc l'efficacité du pompage optique. Il est donc possible de maximiser le taux de polarisation en agissant soit sur la température de l'enceinte d'accumulation (4), soit sur le débit d'injection de mélange à l'aide de la vanne (7). Le débit gazeux dans la cellule de pompage optique (2) doit être adapté au flux lumineux disponible pour polariser les atomes d'hélium-3. Il détermine alors la puissance de réfrigération nécessaire à la liquéfaction. L'échelle de correspondance pour ces trois quantités est la suivante :

1 W de puissance laser permet de polariser correctement jusqu'à 10¹⁸ atomes d'hélium-3 par seconde, ce qui correspond à environ 0,75 litres par seconde d'un mélange gazeux à 4% sous un torr, et qui produit 1,3mm³ par seconde de mélange liquide à 4%. La liquéfaction du mélange à ce rythme dégage une chaleur latente de 4 millijoules par seconde, puissance aisément évacuée par le système de réfrigération. Le temps ultérieurement nécessaire à l'évaporation du liquide accumulé sera négligeable si on utilise une puissance de chauffage très supérieure à 4 mW. Au bilan, le rythme de production du gaz dense polarisé n'est limité que par la puissance laser, à 1 bar.cm³/s pour 1 watt.

Il est également possible d'asservir la température de l'enceinte d'accumulation (4) et/ou le débit d'injection de mélange à la mesure du taux de polarisation délivrée par le dispositif (9).

Pendant la phase d'évaporation, on procède à une légère élévation de température, par exemple par apport calorifique à l'aide d'une petite résistance chauffante, et on procède à la fermeture d'une première vanne (10) pour isoler la cellule de pompage optique (2) et à l'ouverture d'une seconde vanne (11) permettant l'injection du mélange polarisé dans un volume de stockage (6) de gaz à forte pression, ou directement dans un embout relié à l'installation d'injection dans un corps creux, ou d'inhalation par un patient.

A titre d'exemple, le tableau ci-après indique la pression de vapeur (en torrs) pour différentes températures et concentrations d'hélium-3 :

| | 3% | 10% | 30% |
|---|---|---|---|
| T=0,9K | 0,82 | 2,1 | 3,6 |
| T=1,1K | 1,8 | 4,5 | 8,3 |
| T=2,3K | 3,7 | 8,4 | / |

Pendant la phase d'évaporation, la pression augmente avec la température T du volume (4). Pour une pression finale de 1 bar, T est de l'ordre de 4K pour des concentrations de quelques pourcents. La pression critique au-delà de laquelle il n'y a plus d'équilibre liquide/vapeur possible est de l'ordre de 2,3 bar pour l'hélium-4. Au-delà, le chauffage dilate le fluide et augmente encore la pression, comme représenté en figure 2.

Un premier mode d'utilisation consiste à évaporer totalement le liquide accumulé dans le réservoir (4) en le réchauffant suffisamment et en libérant la totalité du gaz polarisé ainsi produit.

Un second mode d'utilisation consiste à n'évaporer que partiellement le liquide accumulé. L'hélium-3 étant plus volatil, sa concentration dans la vapeur est supérieure à celle du liquide. Le gaz récupéré à forte pression est donc plus riche en hélium-3 que dans le mélange initialement condensé. Le liquide résiduel subsistant dans le réservoir (4) contiendra bien entendu corrélativement une proportion plus importante d'hélium-4.

Un troisième mode d'utilisation consiste à préalablement extraire de la solution contenue dans le réservoir (4) une fraction importante de l'hélium-4, et à procéder ensuite à l'évaporation du liquide ainsi enrichi. Plusieurs effets thermomécaniques propres à l'hélium superfluide peuvent être utilisés pour isoler de l'hélium-4 : traversée d'une superfuite, effet "heat flush", effet "hevac".

L'ensemble du volume accessible au fluide polarisé doit être soumis à un champ magnétique suffisamment homogène pour ne pas induire de relaxation. Son amplitude est déterminée par la qualité de l'environnement magnétique ambiant: en général, un millitesla suffit. Elle ne doit par ailleurs pas dépasser quelques dizaines de millitestlas pour que le pompage optique reste efficace.

Un centimètre-cube d'hélium liquide évaporé à température ambiante produit 745 centimètres-cube de gaz à pression atmosphérique. Ainsi, par exemple, 4 cm³ de solution accumulée dans l'enceinte (4) donneront un litre de gaz à 3 bar. Si on n'évapore que le tiers de ce liquide, et qu'il est initialement à une concentration de 3%, on récupérera un litre de gaz à un bar avec une concentration de 5,8%.

Une première application concerne l'imagerie RMN des cavités d'un corps creux, par exemple les poumons d'un patient ou d'un animal. Il convient de faire inhaler un mélange gazeux contenant de l'hélium-3 polarisé préparé avec une installation telle que précédemment décrite.

Une deuxième application est l'imagerie des fluides ou tissus organiques ou biologiques où l'hélium-3 polarisé aura diffusé.

Une troisième application est la caractérisation de matériaux ou de corps poreux par mesure RMN, en utilisant l'hélium-3 polarisé comme sonde locale.

## Revendications

1. Installation pour la production d'hélium-3 polarisé en phase vapeur à forte pression, en particulier pour l'imagerie par RMN, l'installation comportant un moyen d'injection d'hélium-3 ou d'un mélange d'isotopes dans une cellule de pompage optique (2), un moyen de liquéfaction du gaz polarisé provenant de la cellule de pompage optique (2) et un réservoir (4) pour le stockage d'hélium-3 polarisé en phase liquide **caractérisée en ce que** le réservoir (4) d'accumulation est refroidissable par un système de refroidissement à une température ajustable entre une température d'accumulation Ta et une température d'évaporation Te et communique alternativement avec la cellule de pompage optique (2) et avec un conduit d'évacuation de l'hélium-3 gazeux à forte pression.

2. Installation pour la production d'hélium-3 polarisé en phase vapeur selon la revendication 1 **caractérisée en ce que** la cellule de pompage optique (2) communique avec la source d'hélium-3 par une vanne (7) à débit réglable.

3. Installation pour la production d'hélium-3 polarisé en phase vapeur selon la revendication 1 ou 2 **caractérisée en ce que** la cellule de pompage optique (2) comporte un moyen de détermination de la polarisation nucléaire.

4. Installation pour la production d'hélium-3 polarisé en phase vapeur selon l'une quelconque des revendications précédentes **caractérisée en ce qu'**elle comporte des moyens d'ajustage du débit de la vanne d'alimentation de la cellule de pompage et/ou de la température Ta du réservoir d'accumulation (4) en fonction de la polarisation nucléaire dans la cellule de pompage optique (2).

5. Installation pour la production d'hélium-3 polarisé en phase vapeur selon l'une quelconque des revendications précédentes **caractérisée en ce qu'**elle comporte des moyens pour permettre l'évaporation totale ou partielle du liquide polarisé accumulé dans le réservoir (4), sous la forme d'un gaz à forte pression.

6. Installation pour la production d'hélium-3 polarisé en phase vapeur selon l'une quelconque des revendications précédentes **caractérisée en ce que** la cellule de pompage optique (2) est excitée par un laser émettant une raie d'une longueur d'onde de 1083 nanomètres.

7. Procédé pour la production d'hélium-3 polarisé en phase vapeur consistant à injecter dans une cellule de pompage optique (2) de l'hélium et de stocker l'hélium-3 polarisé en phase liquide dans un réservoir de stockage **caractérisé en ce que** le gaz polarisé provenant de la cellule de pompage optique (2) est recueilli dans un réservoir d'accumulation (4) refroidie à une température ajustable entre une températue Ta d'accumulation de l'hélium et une température Te d'évaporation de l'hélium.

8. Procédé pour la production d'hélium-3 selon la revendication 7 **caractérisé en ce que** l'on injecte dans la cellule de pompage optique (2) un mélange d'hélium-3 et d'hélium-4.

9. Procédé pour la production d'hélium-3 selon la revendication 8 **caractérisé en ce que** ledit mélange contient entre 3 et 30% d'hélium-3.

10. Procédé pour la production d'hélium-3 selon l'une quelconque des revendications 6 à 9 **caractérisé en ce que** l'on ajuste le débit et/ou la température du réservoir (4) refroidie en fonction de la polarisation nucléaire mesurée dans la cellule de pompage optique (2).

11. Procédé pour la production d'hélium-3 selon l'une quelconque des revendications 6 à 10 **caractérisé en ce que** la délivrance d'hélium-3 polarisé s'effectue par apport calorifique ou réservoir (4) refroidi qui évapore le liquide accumulé.

12. Procédé pour la production d'hélium-3 selon l'une quelconque des revendications 6 à 11 **caractérisé en ce que** l'on procède à une évaporation-partielle seulement du liquide polarisé contenu dans le reservoir (4) afin d'enrichir le taux d'hélium-3 délivrée par l'installation.

13. Procédé d'imagerie d'un corps comportant une étape consistant à faire pénétrer un mélange gazeux polarisé dans ledit corps **caractérisé en ce qu'**il comporte une étape préalable de stockage d'hélium-3 polarisé dans un réservoir d'accumulation (4) refroidi a une température ajustable entre une température d'accumulation Ta et une température d'évaporation Te, ledit réservoir communiquant alternativement ave une cellule de pompage optique (2) et avec un conduit d'évacuation de l'hélium-3 gazeux.

## Patentansprüche

1. Einrichtung zur Produktion von polarisiertem Hochdruck-Helium-3 in Dampfphase, insbesondere für die NMR-Bilddarstellung, wobei die Einrichtung ein Mittel zum Einblasen von Helium-3 oder eine Isotopenmischung in eine optische Pumpzelle (2), ein Mittel zum Verflüssigen von polarisiertem Gas aus der optischen Pumpzelle (2) und einen Speicher (4) zum Speichern von polarisiertem Helium-3 in Flüssigphase umfasst, **dadurch gekennzeichnet, dass** der Pufferspeicher (4) von einem Kühlsystem auf eine Temperatur zwischen einer Puffertemperatur Ta und einer Verdunstungstemperatur Te abgekühlt werden kann und abwechselnd mit der optischen Pumpzelle (2) und mit einem Rohr zum Abführen des gasförmigen Hochdruck-Helium-3 in Verbindung steht.

2. Einrichtung zur Produktion von polarisiertem Helium-3 in Dampfphase gemäß Anspruch 1, **dadurch gekennzeichnet, dass** die optische Pumpzelle (2) mit der Helium-3-Quelle über ein einstellbares Drosselventil (7) in Verbindung steht.

3. Einrichtung zur Produktion von polarisiertem Helium-3 in Dampfphase gemäß Anspruch 1 oder 2, **dadurch gekennzeichnet, dass** die optische Pumpzelle (2) ein Mittel zur Bestimmung der Kernpolarisierung umfasst.

4. Einrichtung zur Produktion von polarisiertem Helium-3 in Dampfphase gemäß einem der vorhergehenden Ansprüche, **dadurch gekennzeichnet, dass** sie Mittel zum Einstellen der Flussrate des Zufuhrventils der Pumpzelle und/oder der Temperatur Ta des Pufferspeichers (4) in Abhängigkeit von der Kernpolarisierung in der optischen Pumpzelle (2) umfasst.

5. Einrichtung zur Produktion von polarisiertem Helium-3 in Dampfphase gemäß einem der vorhergehenden Ansprüche, **dadurch gekennzeichnet, dass** sie Mittel umfasst, um die vollständige oder teilweise Verdunstung der in dem Speicher (4) gepufferten polarisierten Flüssigkeit in Form von Hochdruckgas zu ermöglichen.

6. Einrichtung zur Produktion von polarisiertem Helium-3 in Dampfphase gemäß einem der vorhergehenden Ansprüche, **dadurch gekennzeichnet, dass** die optische Pumpzelle (2) von einem Laser angeregt wird, der einen Strahl mit einer Wellenlänge von 1083 Nanometer abgibt.

7. Verfahren zur Produktion von polarisiertem Helium-3 in Dampfphase, bestehend aus dem Einblasen des Heliums in eine optische Pumpzelle (2) und dem Speichern des polarisierten Helium-3 in Flüssigphase in einem Speicherbecken, **dadurch gekennzeichnet, dass** das polarisierte Gas aus der optischen Pumpzelle (2) in einem Pufferspeicher (4) gesammelt wird und auf eine einstellbare Temperatur zwischen einer Helium-Puffertemperatur Ta und einer Helium-Verdunstungstemperatur Te abgekühlt wird.

8. Verfahren zur Produktion von Helium-3 gemäß Anspruch 7, **dadurch gekennzeichnet, dass** eine Mischung aus Helium-3 und Helium-4 in die optische Pumpzelle (2) eingeblasen wird.

9. Verfahren zur Produktion von Helium-3 gemäß Anspruch 8, **dadurch gekennzeichnet, dass** die Mischung von 3 bis 30 % Helium-3 enthält.

10. Verfahren zur Produktion von Helium-3 gemäß einem der Ansprüche 6 bis 9, **dadurch gekennzeichnet, dass** die Flussrate und/oder die in Abhängigkeit von der in der optischen Pumpzelle (2) gemessenen Kernpolarisierung abgekühlte Temperatur des Speichers (4) eingestellt wird.

11. Verfahren zur Produktion von Helium-3 gemäß einem der Ansprüche 6 bis 10, **dadurch gekennzeichnet, dass** die Zulieferung von polarisiertem Helium-3 durch Wärmezufuhr zu dem abgekühlten Speicher (4) erfolgt, der die gepufferte Flüssigkeit verdunsten lässt.

12. Verfahren zur Produktion von Helium-3 gemäß einem der Ansprüche 6 bis 11, **dadurch gekennzeichnet, dass** eine Teilverdunstung nur der in dem Speicher (4) enthaltenen polarisierten Flüssigkeit erfolgt, um den von der Einrichtung abgegebenen Helium-3-Gehalt anzureichern.

13. Verfahren zur Bilddarstellung eines Körpers, umfassend einen Schritt des Eindringenlassens einer polarisierten gasförmigen Mischung in den Körper, **dadurch gekennzeichnet, dass** es einen Schritt vor dem Speichern des polarisierten Helium-3 in einem Pufferspeicher (4) umfasst, der auf eine Temperatur abgekühlt ist, die zwischen einer Puffertemperatur Ta und einer Verdunstungstemperatur Te einstellbar ist, wobei der Speicher abwechselnd mit der optischen Pumpzelle (2) und einem Rohr zum Abführen des gasförmigen Helium-3 in Verbindung steht.

## Claims

1. A apparatus for producing high pressure vapor phase polarized helium-3, in particular for NMR-imaging, the apparatus comprising a means for injecting helium-3 or an isotope mixture into an optical pumping cell (2), a means for liquefying polarized gas from the optical pumping cell (2), and a reservoir (4) for storing liquid phase polarized helium-3, **characterized in that** the accumulation reservoir (4) can be cooled down by a refrigeration system to a temperature between an accumulation temperature Ta and an evaporation temperature Te and alternately links to the optical pumping cell (2) and to a duct for discharging high pressure gaseous helium-3.

2. The apparatus for producing vapor phase polarized helium-3 according to claim 1, **characterized in that** the optical pumping cell (2) links to the helium-3 source through a variable flow valve (7).

3. The apparatus for producing vapor phase polarized helium-3 according to claim 1 or 2, **characterized in that** the optical pumping cell (2) comprises a means for determining nuclear polarization.

4. The apparatus for producing vapor phase polarized helium-3 according to any of the preceding claims, **characterized in that** it comprises means for adjusting the flow rate of the supply valve of the pumping cell and/or the temperature Ta of the accumulation reservoir (4) depending on the nuclear polarization in the optical pumping cell (2).

5. The apparatus for producing vapor phase polarized helium-3 according to any of the preceding claims, **characterized in that** it comprises means for allowing total or partial evaporation of the polarized liquid accumulated in the reservoir (4) as high pressure gas.

6. The apparatus for producing vapor phase polarized helium-3 according to any of the preceding claims, **characterized** the optical pumping cell (2) is energized by a laser emitting a ray at a wavelength of 1083 nanometers.

7. A method for producing vapor phase polarized helium-3 comprising the steps of injecting helium into an optical pumping cell (2) and storing the liquid phase polarized helium-3 in a storage reservoir, **characterized in that** the polarized gas from the optical pumping cell (2) is collected in an accumulation reservoir (4) cooled down to a temperature adjustable between a helium accumulation temperature Ta and a helium evaporation temperature Te.

8. The method for producing helium-3 according to claim 7, **characterized in that** a mixture of helium-3 and helium-4 is injected into the optical pumping cell (2).

9. The method for producing helium-3 according to claim 8, **characterized in that** said mixture contains from 3 to 30 % of helium-3.

10. The method for producing helium-3 according to any of claims 6 to 9, **characterized in that** the flow rate and/or the temperature of the reservoir (4) cooled down depending on the nuclear polarization measured in the optical pumping cell (2) is adjusted.

11. The method for producing helium-3 according to any of claims 6 to 10, **characterized in that** the delivery of polarized helium-3 is performed by heat supply to the cooled reservoir (4) which evaporates the accumulated liquid.

12. The method for producing helium-3 according to any of claims 6 to 11, **characterized in that** partial evaporation is performed of the polarized liquid only contained in the reservoir (4) in order to enrich the rate of helium-3 delivered by the apparatus.

13. A method for imaging a body comprising a step of entering a polarized gaseous mixture into said body, **characterized in that** it comprises a step prior to storing polarized helium-3 in an accumulation reservoir (4) cooled down to a temperature adjustable between an accumulation temperature Ta and an evaporation temperature Te, said reservoir linking alternately to the optical pumping cell (2) and a gaseous helium-3 discharge duct.
